# EUROPEAN PATENT APPLICATION

(11) **EP 0 756 879 A2**
(43) Date of publication of application: **05.02.1997**
(21) Application number: 96112246.2
(22) Date of filing: 30.07.1996
(51) Int. Cl.: A61N 1/40

(54) **Electromagnetic shields for thermotherapy**

(30) Priority: 31.07.1995 IT RM950532
(71) Applicant: Franconi, Cafiero, 00184 Roma (IT)
(72) Inventor: Franconi, Cafiero, 00184 Roma (IT)

(57) **Abstract**

The shields concern all short-wave or microwave electromagnetic applicators for thermotherapy in oncology, physical medicine, cosmetology, urology, veterinary medicine and pharmacokinetics, to produce improved heating efficiency and penetration, improved treatment comfort and lower electromagnetic risk to patient's critical organs and to operators. Shields envelopes applicators and parts of body regions producing EM enclosures of containment of radiation leakages. External hood shields block lateral and backward leakages. Regional shields block also leakages through the exposed tissues. Inner shields trap spurious applicators electric fields keeping them away from overheating the patient's adipose. The passive shields protect adjacent critical organs from the treatment fields. The regional shield (26) envelopes both applicator (3) and body region (2) encompassing target (1) heated with beam (10) through the air-gap (8) and establishing the EM enclosure (27) of containment of the EM energy diffused by (3), which blocks the dispersion of the lateral beam fraction (12) through (8) and of the beam fraction (11) that crosses target (1).

## Description

Hyperthermia (HT) is a promising therapeutic modality in oncology for selectively killing cancerous cells in living bodies. Diathermy (DT) is administering heat to tissues in physical medicine and in rehabilitation and has proved useful in treating musculature of superficial and subcutaneus tissues and joints, and in improving collagen extensibility and joint rigidity, pain and muscular spasm relief, and in the resolution of infiltrations, edemas and essudates. Analogous results have been obtained in veterinary medicine. Heat is also employed in rehabilitation treatments in cases of benign prostatic hyperplasia. Further clinical applications of heat concerns the modulation of pharmacokinetic effects and adipose layer dispersion in cosmetology.

The invention concerns all DT and HT clinical treatments in which heat is administered with electromagnetic (EM) means for treating humans and animals. A wide field which is referred to with the comprehensive term of Thermo-Therapy (TT), with the invention aimed to obtain the following advantages: i) a substantial reduction of the EM risk for operators and patients by helping to keep their exposition within safe limits and allowing practical and convenient treatments in non-shielded rooms; ii) improvement in tissue heating efficiency; iii) improving the penetration potential of the treatment heating field and decreasing the risk of overheating the patient's adipose, and iv) limiting the risk to critical organs during TT treatments. The invention applies to all types of prior art HT and DT applicators employing EM energy either at microwaves in the nominal 300-2500 MHz frequency range, hereinafter referred to as MW, or at shortwaves in the nominal 6-300 MHz frequency range, hereinafter referred to as SW, which employ a large variety of heating principles, as partially reported in many reviews *(see: C. Franconi, "Hyperthermia heating technology and devices", in Physics and Technology of Hyperthermia*, *S.Field and C.Franconi eds., pp.80-122, Martinus Nijoff Publ. 1987).*

In the prior art SW capacitive method, heating currents are produced by electric fields (E-fields) from electrodes lying on the body surface through interposed gaps filled with boluses of conductive water. In the prior art inductive SW method, heating currents are induced through air gaps in the tissues by the magnetic fields (H-fields) generated by the radiative structure of the applicator. However, all these prior art SW applicators leak out backward and laterally stray EM radiation. The prior art MW applicators consist of distributed-constant power resonators (tank circuits), of size comparable with the wavelenght, which are fed through shielded coaxial cables. In the 600 to 2450 MHz range, dipole antennae are employed. DT dipole antennae are provided with a "C" type of reflector which helps to focussing the MW beam on the target. Propagation mode waveguides in the 400-2450 MHz MW range irradiate through an aperture whose size is related to the radiation wavelenght. and are filled with dielectric material to reduce their electric dimension to the target size. Dielectric waveguides are usually coupled to the tissue through a gap provided of a conductive water bolus. Evanescent mode, air-filled waveguide applicators are operated in the MW as well as in the SW range, and may be dimensioned to the target without dielectric filling and are coupled to the tissues through air gaps *(see J. Vrba et al., IEEE Trans. BME, Vol. 40, pp. 397-407, 1993).* Composite heating fields are delivered by prior art hybrid SW or MW applicators in which the H-field of an inductive structure integrated in the radiating aperture of air-filled, evanescent-mode waveguides is superposed to the modal field of the waveguides *(US Patent* # *5,186,181).* A further prior art MW applicator consists of a fixed, small size dielectric filled capsule hosting an inductive MW current-sheet radiating structure coupled through a water bolus *(R.H. Johnson et al., Phys. Med. Biol., Vol.35, 761-779, 1990*). Microstrip technology prior art SW and MW applicators consist of either dipoles, or patch resonators of a large variety of shapes and combinations, printed on substrates of suitable dielectric permittivity and magnetic susceptibility to produce compact and sometimes flexible applicators *(see also C. Franconi, loc. cit..).*

The invention concerns shielding methods and devices for the optimization of the TT treatments through the use of EM shielding means on leaking multiple or single prior art SW or MW applicators, be them either inductive or capacitive SW devices, either air filled or dielectric filled waveguides which are operated either in the progressive or in the evanescent mode, waveguide hybrids, dipoles, microstrip or stripline applicators, or applicators derived from these by modifications, integrations and combinations, to help delivering with maximum efficiency and minimum risk the EM energy to tissue targets of any shapes and sizes in either local, or regional or whole body treatments, and maximum comfort and penetration, together with details of their embodiments which fall also within the scope of the invention. Furthermore, the invention includes employing said EM shielding means for the passive shielding of the organs or parts of the body from the exposure to the EM fields produced by TT applicators. The foregoing and other objects and advantages of the present invention will appear in the following description which is referred to in the accompanying drawings in which preferred embodiments and best modes of the invention are illustrated, and in which like numerals refer to like parts. It will be apparent to any TT expert , that modifications, integrations or combinations of the illustrated embodiments fall within the scope of the invention, and that the cited examples of preferred embodiments are not intended to represent limitations on the scope of the invention, which is, instead. susceptible of apparently very different embodiments which fall also within the scope of the invention.

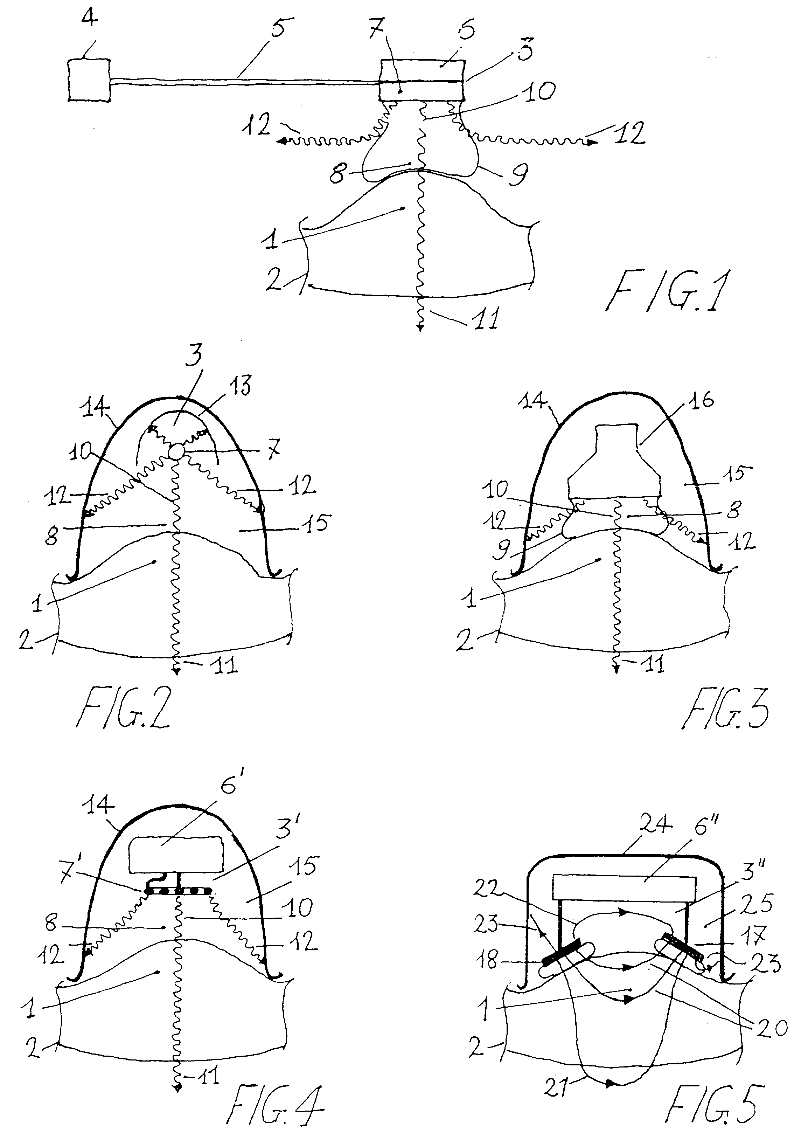

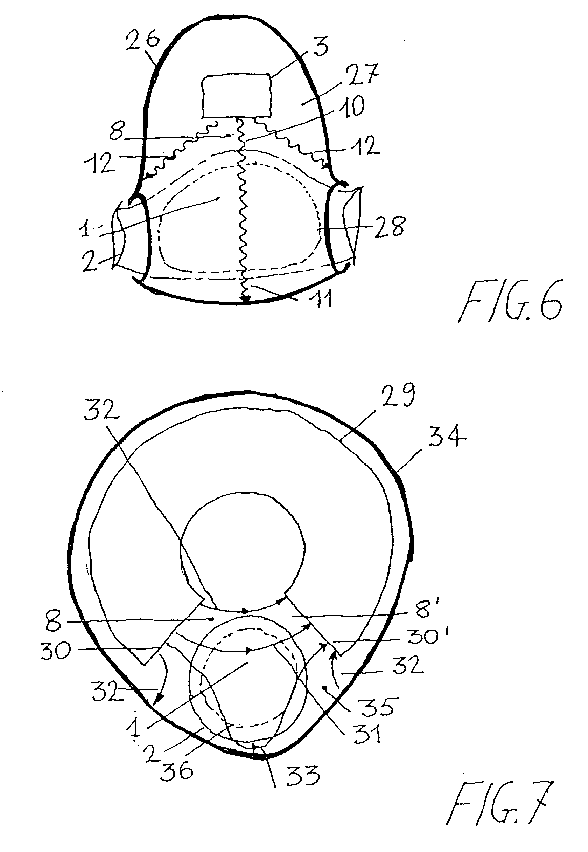

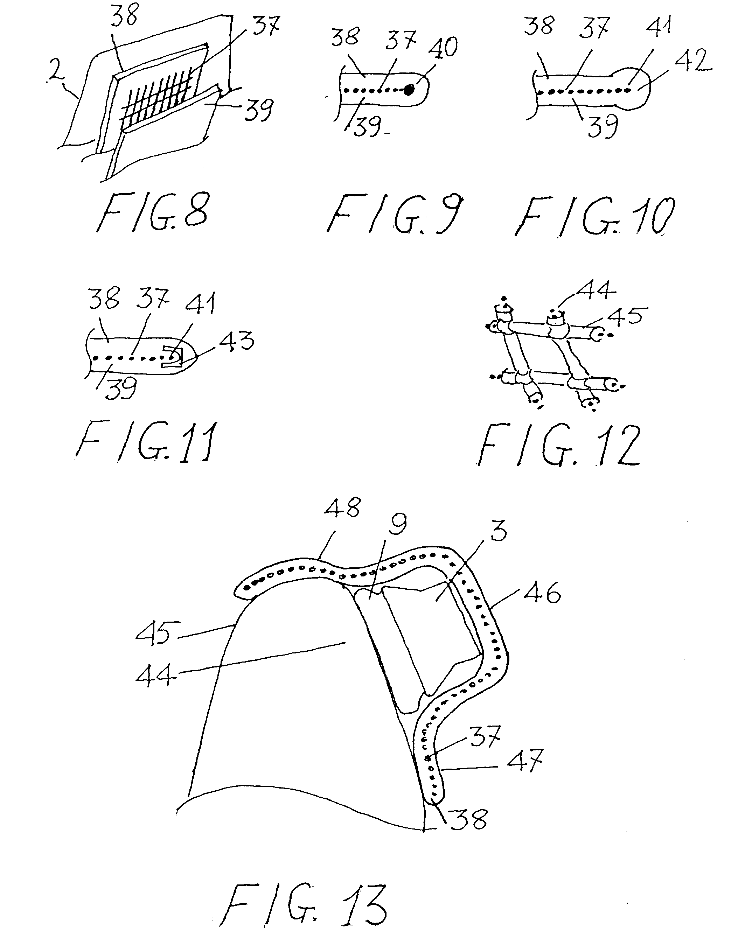

**FIG. 1** shows schematically a prior art TT system in which some functional parts are cut away. The tissue target (1) is embedded in the body region (2) and heated by the applicator (3) fed from the external power generator (4) which is providing EM energy at either SW or MW operating frequencies via the transmission means (5) connected to (3) through the matching and tuning network (6) integrated into (3). Applicator (3) is a tank circuit comprising the radiating structure (7) tuned to the operating frequency from the tuning circuit of network (6), the latter including the circuit for matching the tank circuit to the transmission line (5). The EM energy beam generated by (7) flows through the aperture of (3) and the gap (8), the latter which may be filled with water bolus (9). The fraction (10) of the EM energy beam incident over the region (2) is partially absorbed by the the tissue (1) and emerges with reduced intensity as the leaking beam (11). Waveguide applicators, either air or dielectric filled or hybrid, exhibit a narrower radiation lobe than dipoles. However, on account of the rigid and flat guide aperture, the waveguide gap may have a non-uniform thickness through which a part of the energy radiated by (7) is dispersed laterally. Thus, according to the effective radiation lobe of (3), part of the EM energy from (7) flows through part of the aqueous bolus (9) and leaks out as lateral radiation beam (12).

With the aim of optimizing the advantages of the invention, the leakage of the EM energy in the environment, thereinafter referred to also as dispersion, has to be avoided. The level of dispersion of the SW or MW prior art TT applicators is reduced with the use of the EM shielding methods according to the invention, some preferred embodiments of which are illustrated in the **FIG.s 2-7**, which help also to improve the heating efficiency. **FIG.2** shows part of a preferred shield embodiment for applicator (3) according to the invention, consisting of the dipole radiating structure (7) provided of reflector (13). The shield (14) according to the invention loosely wraps, or envelopes, radiator (7) and reflector (13) together, to impede the beams (12) to leak sideways through gap (8). A preferred embodiment of shield (14) consists of a conductive shielding body, which is shaped to encompass both antenna (7) and reflector (13), to which preferably flexible conductive shielding flaps are attached either permanently, or through conductive mechanical couplings according to practical needs, for extending the resulting peripheral shield conductive walls to adhere all around the patient's skin to provide a partial containment of the EM energy inside the resulting non-perfect EM enclosure (15) characterized by the perfectly shielding wall of (14) and by the non-perfectly shielding tissue (1), the latter crossed by the longitudinal beam (11) which emerges in the environment with an intensity depending on the absorption power of (1). This type of non-perfect shield according to the invention is thereinafter referred to as hood shield . **FIG.3** shows a preferred embodiment with parts cutaway of a hood shield (14) according to the invention which forms the non-perfect shielding enclosure (15) limiting the substantially lateral dispersion of the SW, or MW, dielectric-filled prior art waveguide applicator (16) provided of a water bolus in the gap (8). The SW applicator (3') of **FIG.4** is a shielded version according to the invention of a prior art inductive applicator. The non-perfect shielding enclosure (15), formed by the hood shield (14), wraps applicator (3') which carries the spiral (pancake) or any other inductive coil radiating structure (7') consisting of single or multiple loops coupled through the air-gap (8), and tuned and matched by the substantially capacitive network (6'). The SW capacitive applicator(3") of **FIG.5** is a shielded version according to the invention of the prior art 2-electrode SW capacitive applicator, in which the electrodes (17) and (18), each provided with integral water bolus, are matched and tuned by the substantially inductive network (6"). The current lines (20) cross the water boluses and the tissue (2), whilst the pathway of lines (21) is partially out of the tissue (2) and disperse part of the energy. Lines (22) and (23) cross the air and the water bolus, respectively, and disperse all their EM energy backward and sideways. The hood shield (24) according to the invention generates the non-perfect cavity (25) which limits both lateral and backward dispersion. It will appear evident to any expert, that within the scope of the invention to any prior art TT applicator or to any applicator derived from them by modifications, integrations, or combinations and operated at any SW or MW frequency, can be associated a hood shield of non-critical shape and dimensions modelled over the size (and shapes) of the applicators, and with its periphery conforming to the surface of any anatomic site in body regions also of complex conformation, in order to generate non-perfect enclosures which help to eliminate the lateral and backward dispersion of the EM energy.

**FIG. 6** shows a preferred embodiment of a regional shield according to the invention with parts cutaway in which both the region (2), which includes the tissue target (1), and the generic applicator (3) with the generic radiating structure (7) are wrapped up in a loose and non-critical way with integral shield (26), hereinafter referred to as regional shield, to produce the quasi-perfect shielding enclosure (27) exhibiting a quasi total containment of the EM field, with the exception of the leakage of EM energy through the lateral tissues of the region which cannot be shielded. On account of the smaller dispersion, the sub-region (28) inscribed within the boundary of the regional shield (26) is exposed to an EM field distribution of higher density reaching an higher heating efficiency. **FIG.7** shows the cross-section of the SW inductive, open-mode toroidal applicator (29). The radiative structure consists in the treatment ports (30) and (30') from which an high density and uniform H-field fluxtube comes out and comes in, respectively, and impinges on region (2) releasing heating currents (not shown in **FIG.7**) within the target (1) through air gaps (8) and (8') *(US pat. #,5,099,756).*

Fluxlines such as (31) cross through the target with the highest efficiency. Fluxlines such as (32) from port (30) enter port (30') and totally disperse their energy without crossing the target. The fluxlines such as (33) have their pathway partially outside tissue (2) and partially disperse their energy. The preferred regional shield embodiment (34) according to the invention wraps together applicator (29) and region (2) forming the quasi-perfect shielding enclosure (35), and heat the sub-region (36) with higher efficiency on account of the containment of the leaking energy from both the above H-field lines and the E-field lines (not shown) in the central empty zone of (29) generated by (29) itself. However, there are limits to the maximum size of the shields according to the invention, particularly for the regional ones. In fact, their size should not reach values comparable with the radiation wavelength to avoid steady state resonances in the tissues within the shielding enclosures, which might produce interferences and hot spots in the tissues, keeping into account that the dielectric properties and the thickness of the tissue layers modify the wavelength of the EM radiation. Since the human body is built up of quasi-cylindrical segments, about which efficient shields of congruent symmetry may easily be wrapped, it will appear evident to any expert that regional shields according to the invention may be embodied in any shapes to give quasi-perfect shielding enclosures by wrapping any type of prior art EM applicator heating any site on body regions also of complex configuration. It will also appear evident to any EM expert that on any anatomic site of any region of the body, for both humans and animals, it is possible to apply either hood or regional shields, within the limits imposed by morphological restraints. It will further appear evident to any hyperthermia expert that both the preferred shielding methods of **FIG.s 2-7** and their preferred embodiments of **FIG.s 13-15**, and any other embodiments derived from these by integration, modifications and combinations fall also within the scope of the invention, in that they result of practical and convenient use on any anatomic site of any body region, and improve both the heating efficiency and the electric safety of patients and operators during TT treatments either localized, or regional or loco-regional or whole body ones, which might be performed with any electromagnetic applicators exploiting any heating method or technology.

Moreover, it will appear evident to any expert in electromagnetism that all the shields according to the invention, either hood or regional ones, may be embodied with any conductive material capable of shielding at the operating frequency with a thickness adequate with respect to the penetration of the EM waves due to the skin effect. These materials will preferably be flexible and lightweight, and include thin metallic sheets, conductive fabrics, or fabrics coated with conductive paints, or metallic nets made of thin wires or chains with openings smaller than the operating wavelength in order to avoid a significant dispersion. Furthermore, it will appear evident to any electromagnetic TT expert that, according to the conformation of the anatomic site and/or region, shielding embodiments according to the invention include shields which have their flaps or periphery extending more than is required to be classified as hood shields and less than is required to be classified as regional ones, and in any case having variable extensions, shapes and orientations around the applicator.

On account that any shield subject to the EM field radiation exhibits a potential difference between the shield and the body, the EM shields according to the invention are preferably insulated, even though the exceedingly low level of the EM energy stored by them would exclude any electric risk for the patient, whose skin might be at most subject to harmless microsparks when at direct-contact with the shield. This effect would be amplified in the proximity of pointed shield ends (glare effect). Therefore, according to the invention, the conductive shielding materials are separated from the patient's body by a layer of electrically insulating and preferably flexible material, such as natural or synthetic fabrics also coated with electrically insulating materials, sheets of plastic, or of rubber, or of foams, or of any similar insulating materials. Furthermore, the conductive parts of the shield ought to be electrically connected together for more effective shielding actions.

In **FIG.s 8-12**, preferred embodiments according to the inventions are schematically illustrated with parts cutaway made of conductive shielding materials that are protected with insulating materials and that we refer to as insulated shields. In **FIG.8** a preferred embodiment is schematically illustrated of part of an insulated shield in which the shield (37) is embodied by a thin conductive layer sandwiched between the insulating layer (38) from the patient's side and the insulating layer (39) from the operator's side. In **FIG.9** a preferred embodiment is schematically illustrated of part of a composite shield in which the cross-section of the border (40) of conductive shield (37) is rounded in such a way as not to present either edges or small radius borders that would locally increase the E-field density. In **FIG. 10**, a preferred embodiment is schematically illustrated of part of an insulated shield in which the thin cross-section shield (37) exhibits the border (41) further insulated by the rounded insulating rim (42) made out by a thickening of the insulating layers (38) and (39) ends. In **FIG. 11** is schematically illustrated with parts cutaway a preferred insulated shield embodiment according to the invention in which the highly disperding small radius or pointed far end border (41) of shield (37) is further insulated by the high dielectric strenght protective layer (43) surrounding the pointed border (41) and embedded within the insulating layers (38) and (39). In **FIG. 12** is schematically illustrated with parts cutaway a preferred insulated shield embodiment according to the invention in which the shield (44) is a metallic net made of either wires or chains electrically interconnected and coated with any technique with tubular layers of insulating materials. It will appear evident to any expert that the thickness of the insulating layer (38) from the patient's side may reach a few millimiters and yet remaining flexible when using plastic insulating materials such as PVC, PTFE, Nylon, etc. that possess adequate dielectric strenght or thickness. It follows that the insulating, non-conducting gap having the tickness of the layer (38) between the shield (37) and the patient's skin is an escaping aperture for the EM field. However, such gaps are too thin with respect to the SW and MW wavelengths to allow a significant dispersion of EM energy.

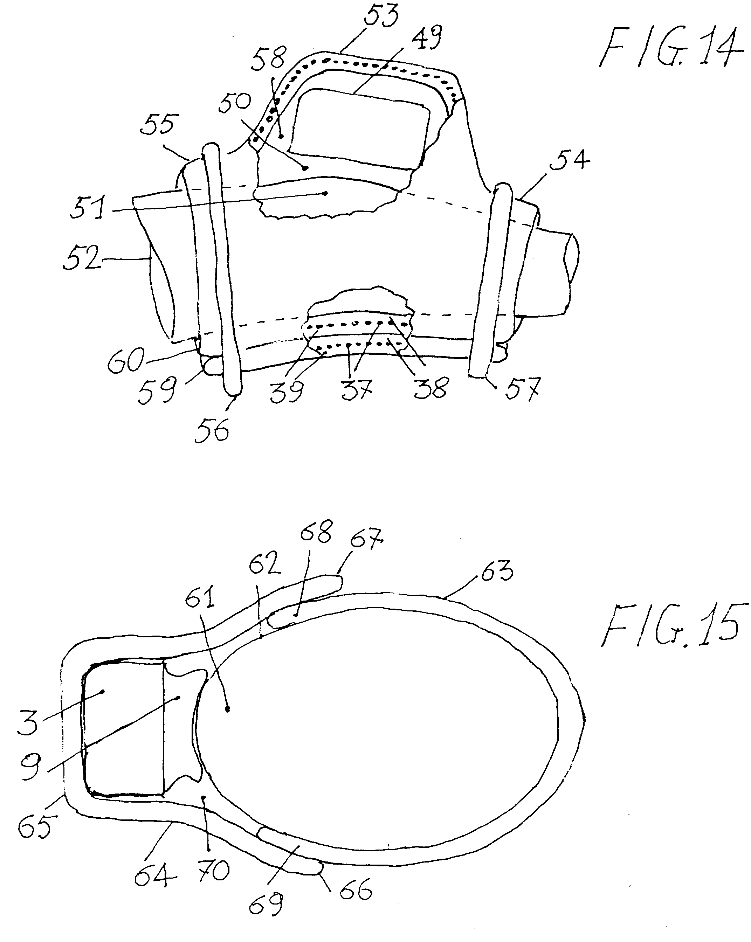

In **FIG.S 13-15** are schematically illustrated with parts cutaway some preferred embodiments of hood and regional insulated shields according to the invention. The **FIG. 13** shows the cross section of parts of a preferred embodiment of a hood insulated shield for applicator (3) heating target (44) in the shoulder region (45) through water bolus (9). This shield presents a central body cap (46) which is modelled over the applicator (3) size. The hood shield consists of the cap shield (46) the rim of which is prolonged with a number of flexible and conformable shielding flaps, of which only (47) and (48) are shown, that are shaped over the many curvatures of the shoulder region (45) to create an efficient EM boundary extended all around the applicator . According to the invention, the insulated shielding cap (46) and the associated peripheral flaps may be constructed either as in one single modelled shield, or else as a plurality of shielding layers partially or totally superposed, with the aim of producing a multilayer efficient shielding hood exhibiting an insulated shielding rim of the required prolongations all around the applicator to perform the required shielding action over the site under treatment. The **FIG. 14** shows a further preferred embodiment of a blanket regional shield for the waveguide applicator (49), either of the evanescent-mode or of the hybrid type, which is heating tissue (51) of the knee region (52) through air gap (50). The shield is a very flexible insulated shielding blanket of rectangular shape, the long side of which wraps together applicator (49) and the quasi-cylindrical knee region, and exhibits a lump due to the applicator, while the two side flaps (54) and (55) are folded over to adhere to the quasi-cylindrical surface of region (52) and kept in place with the help of straps (56) and (57). The **FIG. 15** shows a preferred embodiment of a further regional shield for a generic applicator (3) provided of water bolus (9), which is heating the tissue (61) of he hip site of the pelvic region (62). This regional insulated shield, shown with parts cutaway, is made of two main segments. The first one is the rectangular blanket insulated shield (63) with the long side partially wrapping the pelvis (62). The shield segment (64) is an insulated shield with the lump (65) shaped over the applicator, the side flaps of which have their borders (66) and (67) superposed to the borders (68) and (69) of shield (63) to help creating the EM enclosure (70). The longitudinal and other insulated shielding flaps, not shown in **FIG.15**, are modelled after the region (62) longitudinal and other profiles completing the peripheral EM boundary to create the quasi-perfect enclosure.

EM boundaries of improved effectiveness according to the invention may be created at the either superposed or facing borders of the same or of different insulated shields by providing an effective mechanical coupling which keeps the shields borders tightly close to each other to leave a thin insulated transverse slot, together with a number of electric connections among the conductive materials of the shields for an effective containment of the EM energy. It will appear evident to any expert, that the electric interconnessions among composite shields may be susceptible of many different embodiments, including single or multiple contacts employing metallic wires or chains, laminar contacts, multiple contacts such as with metallic zippers, etc., while always remaining within the scope of the invention.

People expert in electromagnetism know that the radiating structures of any EM applicators for HT and DT produce "near-fields" at close distances from the radiators and when the radiators are electrically "short" with respect to the wavelength. Near-fields possess a substantial E-field component propagating perpendicularly to the fatmuscle interface of the exposed anatomic site, hereinafter referred to as perpendicular E-field (PEF) component, which is found to be overheating the less conductive superficial adipose with respect to underlaying highly conductive muscle layer. Being all MW and SW applicators operated at very short distances from the body surface in order to exploit high energy density non-diverging beams, and being all radiators substantially "short", the tissues are always exposed to a strong PEF component. This represents an unsolved problem in electromagnetic TT and an high risk of overheating the fat layer producing superficial burns, which strongly limits the applicability of TT treatments, particularly for deep targets. We shall refer to the shields according to the invention for the protection against the effects of the PEF, as PEF or inner shields.

Conductive water boluses are used in clinical TT tratments for both separating the tissue surface from the applicator and dissipate a substantial part of the near-field EM energy. However, boluses are most unpractical and are strongly interfering with the treatment parameters. A preferred embodiment of PEF shield according to the invention for general use with any applicator, hereinafter referred to as comb shield, is of the Faraday cage type, and consists of a flat or site conformed comb of parallel thin conductors, preferably interconnected at one end only, which are implemented with technologies (thin wires, PCB, etc) well known to the people expert in electromagnetism. The comb shield is generally positioned across the radiating aperture of any applicator, with the comb conductors aligned with the direction of the main strong therapeutic E-field of the applicator, as any expert in electromagnetism knows, in order to avoid the generation of strong secondary E-fields and associated strong secondary PEF component.

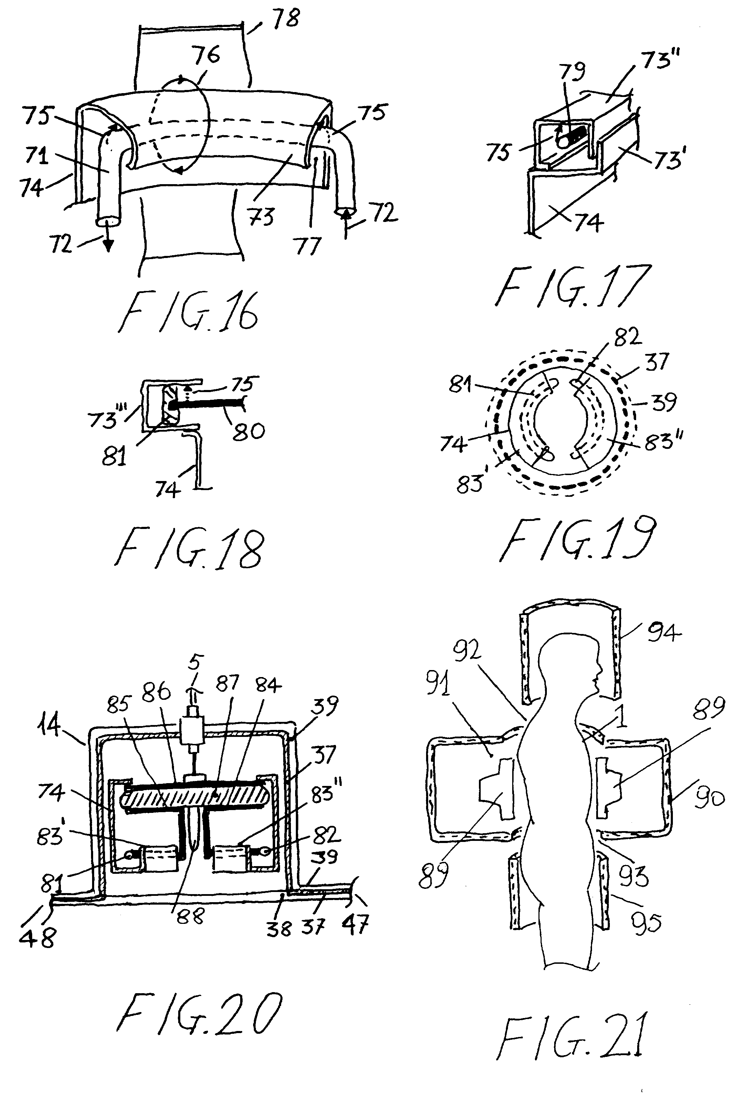

In the inductive SW radiators, high intensity currents flow and potential differences occurs between any points of the radiators which generate high intensity stray E-fields with a substantial PEF component. The related short-range PEF shields according to the invention comprise shielding those active portions of the radiating structures, i.e. the active portions of the inducing current carrying loopsides, which are proximal to the exposed anatomic site, hereinafter referred to as active or proximal loopsides, with inner shields having their shielding borders proximal to the exposed anatomic site, hereinafter referred to as proximal borders, laying close and conformally to the layout of the active loopsides, and generating a substantially capacitive coupling with them. People expert in electromagnetism know that this capacitive coupling helps to concentrate any FEP lines in the close interspace between the proximal shield borders and the active loopside borders, creating a virtual E-field trap. In fact, to an increase of the E-field density in this interspace corresponds a smaller density of FEP field lines impinging perpendicularly on the site surface. **FIG.s 16-19**, schematically illustrate, with parts cutaway, some preferred embodiments according to the invention of FEP shields for the active loopsides of SW inductive applicators. **FIG.16** shows the shaped active portion (71) of a loopside radiator consisting of a tubular conductor in which cooling water (72) flows. The folded proximal border (73) of the inner shield (74) is partially surrounding (71) along all its active length to short the FEP lines such as (75) and deviating them from reaching the exposed tissue (not shown in **FIG. 16**) by substantially shorting them through an effective distributed capacitive coupling all along (71). The current carrying conductor (71), although shielded, can still produce the therapeutic H-field solenoidal lines (76) ouside the shielding wall (73) owing to the longitudinal open slot (77) left all along the shield (73) which is only partially folded. In the background, a portion of the external shield (78), either of the hood or regional type, is illustrated. People expert in electromagnetism know also that the two shielding mechanisms according to the invention are substantially independent; in fact, the external shield (78) and the inner shield (74) are located in different regions of the EM enclosure and are at different electric potential; however, any electric interconnession between them might not allow to separately optimize each shielding action according to the invention. **FIG.17** shows the active portion of the loopside conductive rod radiator (79) and the inner shield (74) which splits into the lower proximal border (73') and the upper proximal border (73") partially surrounding (79) and that are separately constructed and shaped to be easily assembled over the active loopside (79). **FIG.18** shows part of the active loopside radiator (80) that is a current sheet radiator, the side edge of which generates high levels of stray E-fields. This edge is shielded by a "C" profiled conductive shield (73"') conformally following all around the edge of (80), and which is held in place with discrete supports of insulating and losseless materials. The conformal edge shield (73"') is separately constructed and shaped to be easily assembled, and is further electrically connected to the inner shield (74).

**FIG. 19** and **FIG. 20** schematically illustrate, with parts cutaway, how preferred embodiments of the inner and external shields according to the invention easily and usefully apply together to the same prior art SW strip-inductor heating devices *(EPO Patent Application # 95118197.3)* . In **FIG. 19**, the bottom view (tissue side) of the applicator shows two opposed "C" shaped strip-inductor active loopside radiators (81) and (82). According to the invention, the central body of the cylindrical shield (37) and associated external insulating layer (39) are shown together with the cylindrical inner shield (74) terminating with the upper proximal borders (83') and (83") that are shaped conformally to the cylindrical layout of the strip-inductor loopsides (81) and (82), respectively. In **FIG. 20**, the transverse cross section of the a TT applicator is shown including preferred embodiments according to the invention in which the inner shielding effect is optimized with the use of a symmetrizing tuning and matching network which renders more symmetric the SW currents feeding strip inductors (81) and (82) and consequently more symmetric also the stray E-field distribution across them. The tuning circuit embodiment is a symmetric 3-electrode capacitor embodied by a printed circuit board implemented with the lossless dielectric (87), that may be Teflon or other lossless dielectrics of adequate thickness, across which the twin active electrodes (84) and (85) are referred to the central indifferent electrode (86) and are connected to the active strip-inductors (82) and (81), respectively to form the power tank circuit. The feeding of the EM energy to the tank circuit from the feedline (5) may be symmetrized by the use of the variable coupling loop (88) terminating the cable (5), which may be adjusted to provide adequate matching. Such a circuit layout allows to symmetrize and to reduce to an half the stray E-fields along the strip-inductors, and to symmetrize the reduced E-field across the two shields (83') and (83") toward the central (ground) electrode (86). The presence of the external hood shield (14), made of the conductive layer (37) insulated internally and externally by the insulating layers (38) and (39), respectively, which is conformed with a central shielding cap and with peripheral shielding flaps, of which only the (47) and (48) are shown in **FIG.20**, helps to obtain an applicator according to the invention which results optimized with respect to the heating efficiency and penetration, to the EM risk for the operators, and to the comfort and safety for the patients.

It will appear evident to any electromagnetic hyperthermia expert that the preferred PEF shielding methods and embodiments of **FIG.s 16-18** and any embodiments derived from these by integration, modifications and combinations fall also within the scope of the invention, in that they result of practical and convenient use on any anatomic site of any body region, and improve the patient's comfort and the treatment penetration feature of TT treatments, which might be performed with all the prior art applicators and with all applicators derived from these by integrations, modifications and combinations. It will further appear evident to any electromagnetic hyperthermia expert that the two-shield method and their preferred embodiments of **FIG.s 19-20**, and any other embodiments derived from these by integration, modifications and combinations fall also within the scope of the invention, in that they would result of practical and convenient use on any anatomic site of any body region, and are optimized with respect to the heating efficiency, to the EM risk, and to the comfort and penetration features, during TT treatments either localized, or regional or whole body ones, which might be performed with all the prior art MW and SW applicators and with all applicators derived from these by integrations, modifications and combinations.

Finally, it will appear evident to any expert that a complementary usage of the shielding according to the invention, that we shall refer to as passive shielding for brevity, is to shield parts of the body not subject to the treatment from being affected by external EM fields produced by the TT treatment applicators. The **FIG. 21** shows schematically two preferred embodiments of passive insulated shields according to the invention which are shielding the eyes and the reproductive organ of a patient exposed to a TT treatment with the high power multiple annular SW or MW applicator (89) surrounding the patient's trunk (1). The regional shield (90) is modeled after applicators (89) and creates the quasi-perfect enclosure (91) with the openings (92) and (93), due to the anatomic restraints of the region under treatment, through which a not negligeable part of the EM energy leaks out trough the body tissues on account of the large cross-sections of the patient. The passive shields (94) and (95) surround the head and the pelvis of the patient, respectively, and largely attenuate, inside the respective enclosures, the EM energy leaking out from the large openings (92) and (93), respectively. It will appear evident to any expert that composite passive shields may be embodied with a large variety of shapes and sizes to protect any body segment with higher or lower effectiveness according to the anatomic restraints of the region to be protected, while always remaining within the scope of the invention.

## Claims

1. An EM shielding method for thermotherapy treatments of a target tissue of any shape, size and conformation and localized in a generic body region characterized by an anatomic site, said target being exposed to the EM energy beam generated by an EM applicator provided of a radiating structure, said method comprising:
providing a power generator producing EM energy at an operating frequency from about 6 MHz to about 2450 MHz, and feeding said applicator through transmission means;
providing said radiating structure and with a matching and tuning network in order to match said generator to said transmission means;
providing means for positioning said applicator on said site of said region in order that said applicator exposes said target to said energy beam through a gap;
providing external shielding conductive means enveloping said applicator to produce a conductive containment enclosure for said EM energy to prevent the dispersion of said beam of said EM energy into the environment;
providing control means of said external EM shielding means for the optimization of the heating efficiency of said applicator;
providing control means of said external EM shielding means for improving the electric safety of said treatments;
providing internal shielding means for trapping the stray electric field generated by said radiating structure and preventing them to impinge upon the skin of said anatomic site;
providing optimization means for said internal shielding means for improving the comfort of said treatments and the penetration potential of said EM energy beam.

2. A shielding method according to Claim **(1)** in which said means for said efficiency optimization comprises controlling the extension and the conformation of said external shielding means to envelop said applicator with its periphery adhering to the surface of said anatomic site all around said applicator while the conductive walls of said enclosure occlude said gap to prevent said dispersion from occurring backward and sideways.

3. A shielding method according to Claim **(1)** in which said means for said efficiency optimization comprises controlling the extension and configuration of said external shielding means to envelop sad applicator with its periphery adhering to the skin of said body along the boundary of said region encompassing said target, whereby the walls of said enclosure are shielding all said region and contribute to prevent said dispersion of said EM energy beam from occurring backward and sideways through said gap and through said exposed target tissues partially or totally crossed by said EM energy beam.

4. A shielding method according to Claim **(1)** in which said control means for improving said electric safety of said external shielding means comprises covering the whole surfaces of said shielding means with electrically insulating means of adequate dielectric strength whereby the resulting insulated shielding means may be made to adhere in any point to any other point of the surface of said body under electrically safe conditions.

5. A shielding method according to Claim **(1)** in which said control means for optimizing said internal shielding means for improving the comfort of said treatments comprises:
providing said inner shielding means enveloping behind and laterally said radiating structure, said inner shield having its borders proximal to the surface of said site laying close and conformally all around the borders of said radiating structure proximal to the surface of said site, said proximal borders of said inner shield exhibiting a substantially capacitive coupling with said proximal borders of said radiating structure, said capacitive coupling helping to concentrate the spurious electric fields generated by said radiating structure in the interspace between said proximal borders of said inner shield and said proximal borders of said radiating structure;
providing means for optimizing the symmetry and the uniformity of said capacitive coupling all around said portion of said facing radiating structures.

6. A shielding method according to claim **(5)** in which said means for optimizing the symmetry and uniformity of said capacitive coupling all around said proximal borders of said radiating structures comprising:
providing a substantially symmetric circuitry for said matching and tuning network ;
providing said proximal borders of said radiating structures with a substantially symmetric layout;
providing said proximal borders of said inner shield of substantially congruent symmetry with respect to symmetry of said proximal borders of said radiating structures;
providing a substantially symmetric electrical connection to electrically connect said inner shield to said radiating structures through said substantially symmetric network.

7. An EM passive shielding method for protecting a critical organ embedded in a body region subject to an EM risk, said risk arising from the EM fields of frequencies from about 6 MHz to about 2450 MHz that are dispersed by the EM energy beams produced by electromagnetic applicators during thermotherapy treatments of other regions of said body, said passive shielding method consisting of:
providing external shielding conductive means enveloping the whole said region at risk electromagnetic conductive shields exhibiting one or more openings according to the restraints posed by the morphology of said region at risk, said shields forming EM enclosures completely not including said applicators which contributes to prevent the propagation of said EM energy beams inside said region at risk.

8. An external EM shield for safe and efficient thermotherapy treatments of a target tissue embedded in a body region presenting an anatomic site by exposing said tissue to the EM energy beam produced by the radiating structure of an EM applicator, said beam being directed toward said target through a gap, said external shield comprising:
a central shielding body of conductive material extended and conformed to externally envelope said applicator with the exclusion of the aperture crossed by said EM energy beam, said central body being provided of one or more peripherical shielding flaps constructed with conductive materials
constructing said flaps to protrude from said external central shielding body whereby their periphery is conformally extended and adhering to the surface of said body;
means for controlling said extension and conformation of said central shielding body and of said flaps for optimizing the heating efficiency of said applicator.

9. A safe and efficient external hood shield according to claim **8 )** in which said control means of said flaps for said heating efficiency optimization comprises constructing said flaps with said periphery adhering to the surface of said anatomic site all around said applicator in order to prevent a backward dispersion of said EM energy and a lateral dispersion of said EM energy through said gap.

10. A safe and efficient external regional shield according to claim **8)** in which said control means of said flaps for said heating efficiency optimization comprises constructing said flaps with said periphery adhering to the periphery of the surface of said region in order to prevent a dispersion of said EM energy both backward and laterally through said gap as well as the further dispersion of said EM energy beam emerging after having crossed in any direction any portion of said exposed region.

11. A safe and efficient external shield according to claim **8)** in which said means for heating efficiency optimization comprises constructing said central body and/or said flaps with a plurality of shielding layers of configurations congruent to those of both said applicator and said anatomic site or said region, said layers being superposed to minimize the dispersion of said EM energy.

12. A safe and efficient external shield according to claim **8 )** in which said means for heating efficiency optimization comprises constructing said central body and/or said flaps with a plurality of shielding layers, said layers being electrically connected each other to minimize the dispersion of the EM energy.

13. An inner EM shield for improving the comfort and the penetration potential of thermotherapy treatments of a target tissue embedded in a body region presenting an anatomic site by exposing said tissue to the EM energy beam produced by an EM applicator provided of radiating structures, said beam being directed toward said target through a gap, said inner shield comprising:
an inner central shielding body of conductive material extended and conformed to surround said radiative structure of said applicator and exhibiting an opening approximately coinciding with the aperture of said applicator in order to let said EM energy beam to propagate towards said target, the borders of said inner shield opening proximal to the surface of said site lying close and conformally around the borders of said radiating structures proximal to the surface of said site and establishing a distributed capacitive coupling therebetween;
means for controlling said capacitive coupling for improving the uniformity of the electric field concentration between said borders.

14. An inner shield according to claim **13)** in which said applicator is a shortwave inductive one, and said radiating means are coils constructed with conductive materials that are shaped as either a spiral or as single or multiple loops carrying shortwave currents, said coils being constructed with any conformation, configuration and size, said coils laying with their planes oriented substantially parallel to the surface of said body, and said means for controlling said capacitive coupling comprises constructing said inner shield as a conductive inner central body with said proximal borders shaped as preferably flexible flanges partially wrapping said current carrying loops conformally to the symmetry of the layout of said border of said proximal radiating structures.

15. An inner shield according to claim **13)** in which said applicator is a strip-inductor applicator, and said radiating means are coils constructed with conductive materials that are shaped as either spirals or as single or multiple loops carrying shortwave currents, said coils being constructed with any conformation and size, said coils laying with their planes oriented substantially orthogonally to the surface of said body, and said means for controlling said capacitive coupling comprises constructing said inner shield as a conductive inner central shielding body with said proximal borders shaped as preferably flexible flanges partially wrapping said current carrying loops conformally to the symmetry of the layout of said borders of said proximal radiating structures.

16. An inner shield according to claim **13)** in which said applicator is an evanescent-mode air-filled waveguide applicator in which current carrying inductive radiating structures are integrated into the aperture of said waveguide to produce and hybrid applicator, said radiating structures being coils constructed as either single or multiple loops of any conformation, configuration and size and exhibiting any configuration with respect to the conductive aperture rim of said waveguide, said radiating structures being constructed with conductive materials, and said means for controlling said capacitive coupling comprises constructing said inner shield proximal border by providing the rim of said waveguide aperture with conductive flanges partially wrapping and shielding the portion of said current carrying loops proximal to the surface of said site to produce an open shield layout that is conformally following the layout of said current carrying loops proximal to the surface of said site.

17. An inner shield according to claim **13)** in which said applicator is substantially inductive and consists of a toroidal applicator exhibiting either flat or curved, or rigid or flexible treatment ports, and said means for controlling said substantially capacitive coupling comprises constructing said inner shield forward border conformally following the layout of said border of said portion of said treatment ports proximal to the surface of said site and behave as conformal guard rings for said treatment ports.

18. An inner shield according to claim **13)** in which said applicator is substantially inductive and consists of an open-ended metallic capsule hosting radiating means consisting of current sheet radiators of any conformation and size and exhibiting any configuration with respect to the conductive rim of the aperture of said capsule, and said means for controlling said capacitive coupling comprises constructing said inner shield proximal borders by providing the rim of said aperture with conductive flanges partially shielding the edges of said current carrying sheets while conformally following the layout of the said current carrying sheets proximal to the surface of said anatomic site.

19. An inner shield according to claim **13)** in which said applicator is substantially capacitive and consists of two or more capacitive electrodes, and said means for controlling said substantially capacitive coupling comprises constructing said inner shield proximal borders as a guard rings conformally following said proximal borders of each said electrode.

20. An inner shield according to claim **13)** in which said applicator is substantially radiative and consists of antennae made of single or multiple microstrip or strip-line resonators on either flat or curved, rigid or flexible, substrates, and said means for controlling said capacitive coupling comprises constructing said proximal inner shield borders as guard rings to said resonators conformally following the external layout of said proximal borders of said resonators.
